# EUROPEAN PATENT SPECIFICATION

(11) **EP 2 535 066 B1**
(45) Date of publication and mention of the grant of the patent: **05.04.2017**
(21) Application number: 11742263.4
(22) Date of filing: 09.02.2011
(51) Int. Cl.: A61M 1/14, A61M 1/34

(54) **BLOOD PURIFICATION DEVICE**
BLUTREINIGUNGSVORRICHTUNG
DISPOSITIF DE PURIFICATION DU SANG

(30) Priority: 10.02.2010 JP 2010028183
(43) Date of publication of application: 19.12.2012
(73) Proprietor: Nikkiso Company Limited, Tokyo 150-6022 (JP)
(72) Inventor: SUGIOKA, Akira, Makinohara-shi Shizuoka 421-0496 (JP)
(74) Representative: von Hirschhausen, Helge
(86) International application number: PCT/JP2011/052753
(87) International publication number: WO 2011/099520

(56) References cited:
- JP-A- 2 289 259
- JP-A- 2004 313 522
- JP-A- 2005 218 709
- US-B2- 6 916 424

## Description

### Technical Field

The present invention relates to a blood purification apparatus which performs a blood purification treatment using a blood purifier connected to a blood circuit.

### Background Art

Recently, in a dialysis apparatus as a blood purification apparatus, a technique has been suggested which performs priming, reinfusion, and dialysate infusion (emergency dialysate infusion) using a dialysate for being supplied to a dialyzer during dialysis treatment (particularly, an on-line HDF or an on-line HF). For example, Patent Document 1 discloses a dialysis apparatus which includes a dialysate infusing line that has one end connected to a collection port formed in a predetermined part of a dialysate introduction line and the other end connected to a blood circuit (arterial blood circuit or venous blood circuit), and a dialysate infusing pump disposed in the dialysate infusing line. In order to perform the priming, the reinfusion or the dialysate infusion (the emergency dialysate infusion) using the dialysis apparatus, the dialysate in a dialysate introduction line is supplied to the blood circuit (the arterial blood circuit or the venous blood circuit) by driving the dialysate infusing pump.

### Citation List

### Patent Document

Patent Document 1: Japanese Laid-open Patent Publication No. 2004-313522
Patent Document 2: US6916424B2

### Summary of Invention

The invention is defined in the appended claims.

### Technical Problem

However, in the blood purification apparatus of the related art, in order to perform the priming, the reinfusion or the dialysate infusion (the emergency dialysate infusion), there has been a need for a work of connecting one end of the dialysate infusing line to the collection port formed in a predetermined part of the dialysate introduction line. Thus, a worker has needed to visually confirm whether or not the connection is normally performed. For this reason, there has been a concern of human error, such as forgetting of the connection confirmation, being generated.

The present invention has been made under such circumstances, and provides a blood purification apparatus capable of preventing the forgetting of the dialysate infusing line connection by automatically performing the connection confirmation of the dialysate infusing line to the collection port formed in a predetermined part of the dialysate introduction line.

### Solution to Problems

According to aspect 1, there is provided a blood purification apparatus that includes a blood purifier which includes a blood purification membrane and performs blood purification in the blood purification membrane; an arterial blood circuit, a proximal end of which is connected to the blood purifier and in which a blood pump is disposed in the middle thereof; a venous blood circuit, a proximal end of which is connected to the blood purifier; a dialysate introduction line which introduces a dialysate into the blood purifier; a dialysate discharge line which discharges the dialysate from the blood purifier; a dialysate infusing line, one end of which is connected to a collection port formed in a predetermined part of the dialysate introduction line, and the other end of which is connected to the arterial blood circuit or the venous blood circuit; and a dialysate infusing pump which is disposed in the dialysate infusing line and is able to supply the dialysate of the dialysate introduction line to the arterial blood circuit or the venous blood circuit, wherein a closed circuit is formed in a flow route of the dialysate of the dialysate introduction line side including the predetermined part formed with the collection port, and a test process for confirming the connection of the dialysate infusing line to the collection port by measuring a fluid pressure in the closed circuit while driving the dialysate infusing pump is performed.

According to aspect 2, in the blood purification apparatus described in aspect 1, wherein a clamp device capable of opening and closing the flow route thereof is disposed in the dialysate infusing line, and the connection confirmation of the dialysate infusing line and the opening and closing confirmation of the clamp device can be performed in the test process.

According to aspect 3, in the blood purification apparatus described in aspect 1 or 2, wherein a positive pressure is applied to the closed circuit by driving the dialysate infusing pump in reverse rotation in the test process.

According to aspect 4, in the blood purification apparatus described in any one of aspects 1 to 3, wherein the apparatus includes a measurement device capable of measuring the fluid pressure in the closed circuit of the state where the dialysate infusing pump is driven in the test process, and a determination device for determining whether or not the dialysate infusing line is connected normally to the collection port based on the measured value of the measurement device.

### Advantageous Effects of Invention

According to aspect 1, since the closed circuit is formed in a flow route of the dialysate of the dialysate introduction line side including the predetermined part formed with the collection port, and a test process for confirming the connection of the dialysate infusing line to the collection port by measuring the fluid pressure in the closed circuit while driving the dialysate infusing pump is performed, it is possible to prevent the forgetting of the dialysate infusing line connection by automatically performing the connection confirmation of the dialysate infusing line to the collection port.

According to aspect 2, since the clamp device capable of opening and closing the flow route thereof is disposed in the dialysate infusing line, and the connection confirmation of the dialysate infusing line and the opening and closing confirmation of the clamp device can be performed in the test process, it is possible to prevent the forgetting of the clamp device opening operation in addition to the prevention of the forgetting of the dialysate infusing line connection, and it is possible to more reliably perform circulation of the dialysate in the dialysate infusing line.

According to aspect 3, since a positive pressure is applied to the closed circuit by driving the dialysate infusing pump in reverse rotation in the test process, when performing the connection confirmation of the dialysate infusing line to the collection port, it is possible to more reliably confirm a case where the connection is not normal.

According to aspect 4, since the apparatus includes a measurement device capable of measuring the fluid pressure in the closed circuit of the state where the dialysate infusing pump is driven in the test process, and a determination device for determining whether or not the dialysate infusing line is connected normally to the collection port based on the measured value of the measurement device, it is possible to more accurately and automatically perform the connection confirmation of the dialysate infusing line to the collection port.

### Brief Description of Drawings

[Fig. 1] Fig. 1 is a schematic diagram that shows a dialysis apparatus according to an embodiment of the present invention.
[Fig. 2] Fig. 2 is a schematic diagram that shows a state when a test process is performed in the dialysis apparatus.
[Fig. 3] Fig. 3 is a cross-sectional schematic diagram that shows a collection port in the dialysis apparatus.
[Fig. 4] Fig. 4 is a cross-sectional schematic diagram that shows a state where a dialysate infusing line is connected to the collection port.
[Fig. 5] Fig. 5 is a flowchart that shows a control content of the test process in the dialysis apparatus.

### Description of Embodiments

Hereinafter, an embodiment of the present invention will be specifically described with reference to the drawings.

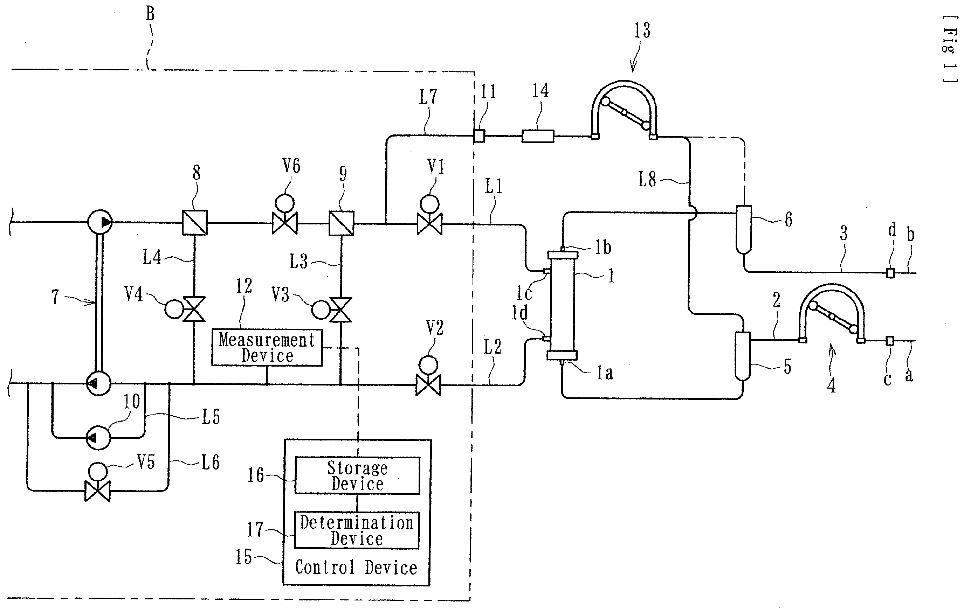

A blood purification apparatus according to the present embodiment is applied to a blood dialysis apparatus. As shown in Fig. 1, the blood purification apparatus mainly includes a blood circuit in which an arterial blood circuit 2 and a venous blood circuit 3 are connected to a dialyzer 1 as a blood purifier, a dialysis apparatus main body B having a dialysate introduction line L1 and a dialysate discharge line L2, a dialysate infusing line L8, a measurement device 12, a dialysate infusing pump 13, and a control device 15.

The dialyzer 1 includes a blood purification membrane (not shown) (although the membrane is a hollow fiber type blood dialysis filtration membrane in the present embodiment, the membrane includes a flat membrane type, blood dialysis membrane and a blood filtration membrane). The dialyzer 1 is formed with a blood introduction port 1a for introducing the blood and a blood delivery port 1b for delivering the introduced blood, is formed with a dialysate introduction port 1c for introducing the dialysate and a dialysate discharge port Id for discharging the introduced dialysate, and purifies the blood by bringing the dialysate into contact with the blood introduced from the blood introduction portion 1a via a hollow fiber.

The arterial blood circuit 2 is mainly constituted by a flexible tube, and one end thereof is connected to a blood introduction portion 1a of the dialyzer 1 to guide the blood collected from a patient's blood vessel into the hollow fiber of the dialyzer 1. In the other end of the arterial blood circuit 2, a connector c capable of attaching an arterial puncture needle a is formed, an arterial air trap chamber 5 is connected to the middle thereof, and a blood pump 4 is disposed. Furthermore, the blood pump 4 is a peristaltic type pump (having a configuration that squeeze the flexible tube when being rotated forward to cause the blood to flow from the arterial puncture needle a in the direction of the blood introduction port la of the dialyzer 1).

The venous blood circuit 3 is mainly constituted by the flexible tube as in the arterial blood circuit 2, and one end thereof is connected to a blood introduction port 1b of the dialyzer 1 to introduce the blood passing through the hollow fiber. In the other end of the venous blood circuit 3, a connecter d capable of attaching a venous puncture needle b is formed, and a venous air trap chamber 6 is connected to the middle thereof. That is, the patient's blood collected by the arterial puncture needle a reaches the dialyzer 1 via the arterial blood circuit 2. After the blood purification is performed, the blood flows though the venous blood circuit 3, and returns back to the body of the patient via the venous puncture needle b, whereby the extracorporeal circulation is performed.

The dialysate introduction line L1 and the dialysate discharge line L2 are connected to the dialysate introduction port 1c and the dialysate discharge port 1d of the dialyzer 1, respectively, and the dialysate introduced to the dialyzer 1 via the dialysate introduction line L1 can be discharged from the dialysate discharge line L2 through the outside of the hollow fiber. An electromagnetic valve V1 and an electromagnetic valve V2 are connected to the middle of the dialysate introduction line L1 and the dialysate discharge line L2, respectively.

Furthermore, a duplex pump 7, which supplies the dialyzer 1 with the dialysate prepared to a predetermined concentration and discharges the dialysate from the dialyzer 1, is connected to the dialysate introduction line L1 and the dialysate discharge line L2, and filtration filters 8 and 9 are connected between the duplex pump 7 in the dialysate introduction line L1 and the electromagnetic valve V1. The filtration filters 8 and 9 filter and purify the dialysate flowing through the dialysate introduction line L1, and bypass lines L3 and L4 for guiding the dialysate so as to bypass the dialysate discharge line L2 are formed from the filtration filters 8 and 9. Electromagnetic valves V3 and V4 are connected to the bypass lines L3 and L4, respectively. Furthermore, an electromagnetic valve V6 is connected between the filtration filter 8 and the filtration filter 9 in the dialysate introduction line L1.

Meanwhile, measurement device 12 capable of measuring the fluid pressure of the dialysate is connected between the connection section with the bypass line L3 and the connection section with the bypass line L4 in the dialysate discharge line L2. Furthermore, the dialysate discharge line L2 is formed with bypass lines L5 and L6 that bypass the duplex pump 7, an ultrafiltration pump 10 for removing the water content from the patient's blood flowing in the dialyzer 1 is disposed in the bypass line L5, and the electromagnetic valve V5 capable of opening or closing the flow route is disposed in the bypass line L6. Furthermore, a closed circuit mentioned below can be formed by arbitrarily and selectively opening and closing the electromagnetic valves V1 to V6.

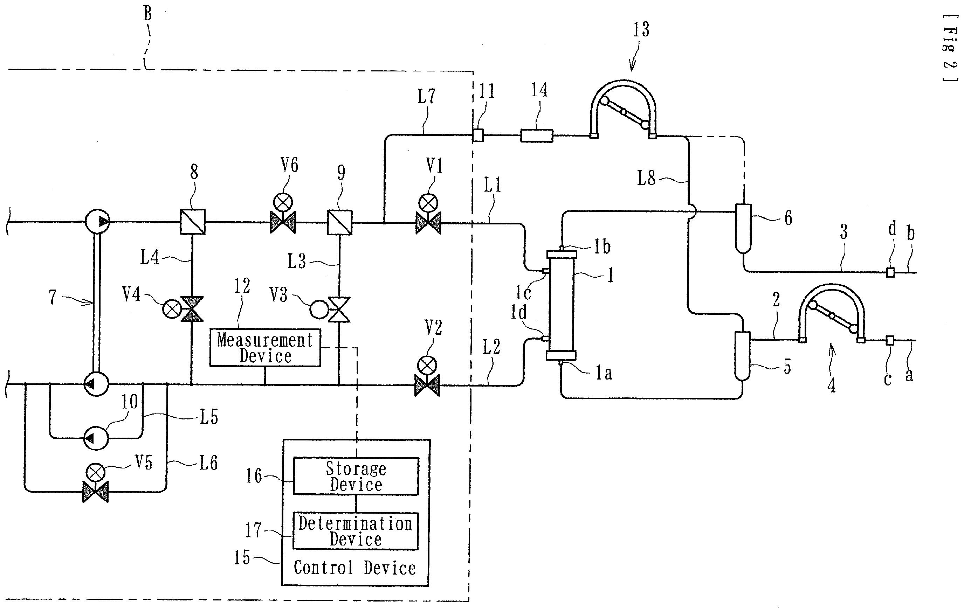

One end of the dialysate infusing line L8 is connected to a collection port 11 formed in a predetermined part (a tip of a branch line L7 branched from the dialysate introduction line L1 in the present embodiment) of the dialysate introduction line L1, and the other end thereof is constituted by a flow route connected to the arterial air trap chamber 5 (or the venous air trap chamber 6 of the venous blood circuit 3 as shown by a two-dot chain line in Fig. 2) of the arterial blood circuit 2.

However, the dialysate infusing line L8 is disposed with clamp device 14 capable of opening and closing the flow route thereof. After the dialysate infusing line L8 is connected to the collection port 11 by a worker, the clamp device 14 is in the closed state until the dialysate is caused to circulate, and the flow route is closed. Moreover, if necessary (during priming, reinfusion, dialysate infusion or the like), the clamp device 14 is in the open state by a worker, and the dialysate introduction line L1 communicates with the blood circuit (the arterial blood circuit 2 or the venous blood circuit 3).

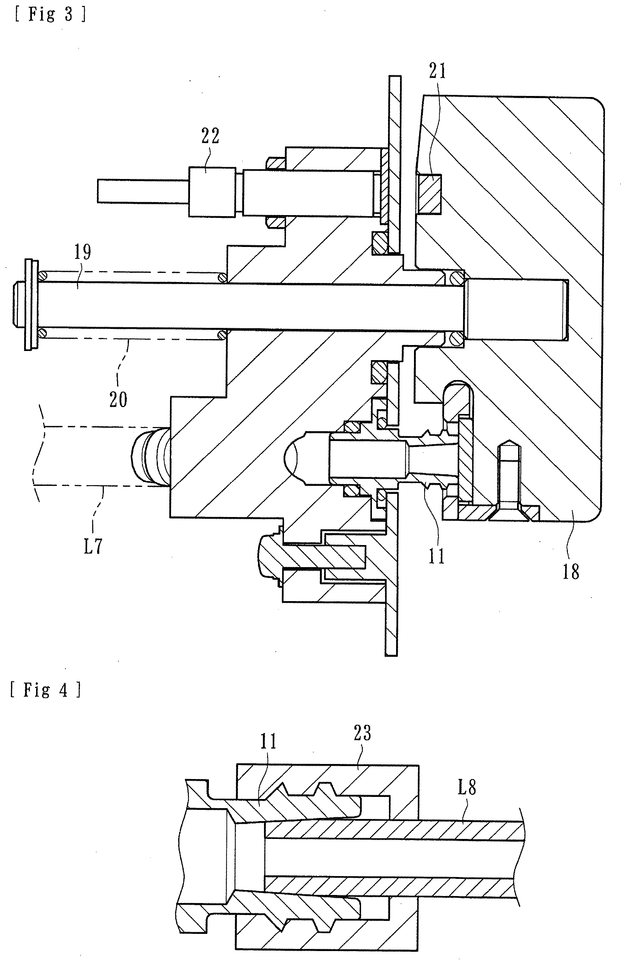

Meanwhile, the collection port 11 is formed by a port formed in the dialysis apparatus main body B, and can be opened and closed by a lid member 18 that can be rotated around a shaft 19, as shown in Fig. 3. Specifically, the lid member 18 can be rotated between a closed position that covers the collection port 11 and an open position that causes the collection port 11 to face outside, and, when in the closed position, can close an opening end of the collection port 11 by the biasing force of a spring 20.

Furthermore, the lid member 18 can be shifted from the closed position to the open position by moving the lid member 18 held in the closed position to right direction of the drawings against biasing force of the spring 20 and rotating the lid member 18 around the shaft 19. In that state, as shown in Fig. 4, one end of the dialysate infusing line L8 is connected to the collection port 11. As shown in Fig. 4, the one end of the dialysate infusing line L8 is formed with a connector member 23. Thus, it is possible to firmly connect the collection port 11 and the one end of the dialysate infusing line L8 by screwing a female screw section formed in the connector member 23 with a male screw section formed in the collection port 11 in a state of fitting the one end into the collection port 11.

Furthermore, in a predetermined part of the lid member 18, a magnet 21 capable of generating a magnetic force is formed, and a lead switch 22 is disposed which is capable of detecting a magnetic force in a position opposed to the magnet 21 and generating an electrical on-off signal when the lid member 18 is in the closed position. As a result, it is possible to detect whether the lid member 18 is in the closed position or the opened position, based on the on signal or the off signal of the lead switch 22.

The dialysate infusing pump 13 is disposed in the dialysate infusing line L8 and is able to supply the dialysate of the dialysate introduction line L1 to the blood circuit (the arterial blood circuit 2 or the venous blood circuit 3). Furthermore, as in the blood pump 4, the dialysate infusing pump 13 is a peristaltic type pump (having a configuration that squeezes the tube constituting the dialysate infusing line L8 when being rotated forward to allow the dialysate to flow), and is able to be rotated forward and reverse.

The control device 15 is formed of, for example, a microcomputer or the like that can control the opening and closing the various electromagnetic valves V1 to V6 disposed in the dialysis apparatus and an actuator of the blood pump 4, the dialysate infusing pump 9 or the like. Particularly, in the present embodiment, the control device 15 is formed with storage device 16, determination device 17 or the like and performs the test process for the connection confirmation of the dialysate infusing line L8 to the collection port 11 and the opening and closing confirmation of the clamp device. The test process is a process for performing the connection confirmation of the dialysate infusing L8 to the collection port 11 and the opening and closing confirmation of the clamp device 14 by measuring the fluid pressure in the closed circuit by the measurement device 12 while driving the dialysate infusing pump 13 in a state where, in the flow route (that is, a branch line L7, the dialysate introduction line L1 and the dialysate discharge line L2 or the flow route of the dialysate in the dialysis apparatus main body B branched from the lines) of the dialysate of the dialysate introduction line L1 including a predetermined part formed with the collection port 11 is formed with the closed circuit.

Herein, the closed circuit in the test process is formed by making the electromagnetic valves V1, V2, V4, V5 and V6 be in the closed state and the electromagnetic valve V3 be in the opened state, and is able to measure the fluid pressure (the fluid pressure of the dialysate) in the closed circuit by the measurement device 12. Furthermore, the closed circuit may be configured so as to include the part formed with the measurement device 12, and, for example, may be formed by making the electromagnetic valves V1, V2 and V5 be in the closed state and the electromagnetic valves V3, V4 and V6 be in the opened state.

The measurement device 12 according to the present embodiment is able to measure the fluid pressure in the closed circuit of the state of driving the dialysate infusing pump 13 in the test process, and is electrically connected to the storage device 16 of the control device 15. The storage device 16 is able to store a measured value (the fluid pressure) measured by the measurement device 12 in the test process, and is connected to the determination device 17. The determination device 17 determines whether or not the dialysate infusing line L8 is connected normally to the collection port 11 or the clamp device 14 is in the opened state, based on the measured value of the measurement device 12 stored in the storage device 16.

That is, in a case where the dialysate infusing line L8 is not connected normally to the collection port 11, when driving (the normal rotation driving or the reverse rotation driving) the dialysate infusing pump 13 in the test process, giving the positive pressure or the negative pressure to the closed circuit and then stopping the dialysate infusing pump 13, since the fluid pressure measured by the measurement device 12 is reduced over time, if the determination device 17 determines a decrease in fluid reduction, it is possible to determine that the connection to the collection port 11 is not normal. Furthermore, in a case where the clamp device 14 is in not the opened state (similarly, a case where the dialysate infusing line L8 is not completely connected to the collection port 11 and the collection port 11 is in the opened state), even when driving (forward rotation driving or reverse rotation driving) the dialysate infusing pump 13 in the test process, the positive pressure or the negative pressure cannot be applied to the closed circuit and the measure value is different from an initial value. Thus, when the determination device 17 determines that state, it is possible to determine that the clamp device 14 is not in the opened state.

In the present embodiment, the positive pressure is applied to the closed circuit by driving the dialysate infusing pump 13 for the reverse rotation in the test process. As a result, when driving the dialysate infusing pump 13 for the reverse rotation to apply a positive pressure, for example, in a case where the fitting of one end of the dialysate infusing line L8 to the collection port 11 is defective, or in a case where screw fitting using the connector member 23 is defective, the one end of the dialysate infusing line L8 is separated from the collection port 11 by the applied positive pressure, the pressure measured by the measurement device 12 is reduced, and thus, the connection defect can be more accurately detected.

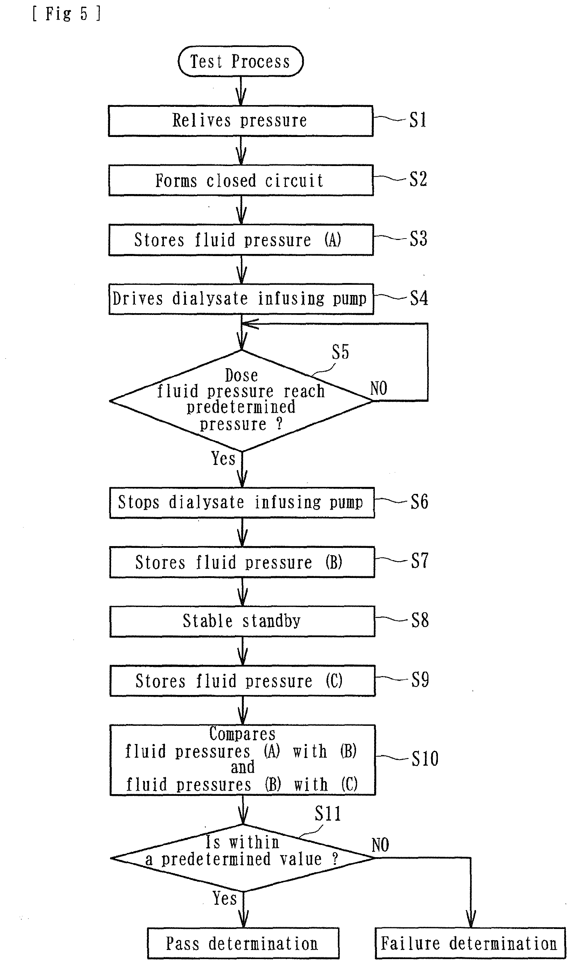

Hereinafter, the control contents of the control device 15 in the test process will be described based on a flowchart of Fig. 5.

Firstly, the pressure is relieved by making the electromagnetic valves V3, V4 and V5 be in the opened state (S1). After that, as shown in Fig. 2, by making the electromagnetic valves V1, V2, V4, V5 and V6 be in the closed state and the electromagnetic valve V3 be in the opened state, the closed circuit is formed (S2), a fluid pressure (A) measured by the measurement device 12 in the state is stored in the storage device 16 (S3), and the dialysate infusing pump 13 is driven (the reverse rotation driving in the present embodiment) (S4). Moreover, it is determined whether the fluid pressure measured by the measurement device 12 reaches a predetermined pressure (S5). Furthermore, in the present embodiment, although whether or not the fluid pressure reaches a predetermined fluid pressure is determined in S5, whether or not a predetermined time elapses or the dialysate infusing pump 13 is rotated by a predetermined amount may be determined. Furthermore, the determinations may be combined and performed.

When it is determined that the fluid pressure measured by the measurement device 12 in S5 reaches a predetermined fluid pressure, the process proceeds to S6, and the dialysate infusing pump 13 is stopped (S6). In that state, the measured value (the fluid pressure) of the measurement device 12 is stored in the storage device 16 and is set to the fluid pressure (B) (S7), and the stable standby is performed for a predetermined time (for example, about 4 seconds) (S8). If the standby time in S8 is set to be longer, the accuracy can be enhanced. Furthermore, the measured value (the fluid pressure) of the measurement device 12 is stored in the storage device 16 and is set to the fluid pressure (C) (S9).

After that, by the determination device 17, the fluid pressure (A) stored in the storage device 16 is compared to the fluid pressure (B), and the fluid pressure (B) is compared to the fluid pressure (C) (S10), it is determined whether or not the pressurization is performed normally based on the comparison of the fluid pressure (A) and the fluid pressure (B), and it is determined whether or not there is leakage based on the comparison of the fluid pressure (B) and the fluid pressure (C) (S11). Furthermore, if it is determined that the pressurization is performed normally and there is no leakage, the result is determined as a pass, and it is recognized that the connection of the dialysate infusing line L8 to the collection line 11 is normal. Meanwhile, if it is determined that the pressurization is not normally performed or there is a leakage, the result is determined as a failure, and it is recognized that the connection of the dialysate infusing line L8 to the collection line 11 is not normal.

According to the configuration mentioned above, the closed circuit is formed in the flow route of the dialysate of the dialysate introduction line L1 side including a predetermined part formed with the collection port 11, and the test process for the connection confirmation of the dialysate infusing line L8 to the collection port 11 is performed by measuring the fluid pressure in the closed circuit while driving the dialysate infusing pump 13. Thus, the connection confirmation of the dialysate infusing line L8 to the collection port 11 is automatically performed, and it is possible to prevent the forgetting of the dialysate infusing line L8 connection.

Furthermore, since the dialysate infusing line L8 is disposed with the clamp device 14 capable of opening and closing the flow route thereof, and the connection confirmation of the dialysate infusing line L8 and the opening and closing confirmation of the clamp device 14 can be performed in the test process, it is possible to prevent the forgetting of the clamp device 14 opening operation in addition to the prevention of the forgetting of the dialysate infusing line L8 connection, whereby it is possible to more reliably perform the circulation of the dialysate in the dialysate infusing line L8.

Furthermore, since a positive pressure is applied to the closed circuit by driving the dialysate infusing pump 13 for the reverse rotation in the test process, when performing the connection confirmation of the dialysate infusing line L8 to the collection port 11, it is possible to more accurately perform the confirmation of a case where the connection is not normal. Furthermore, since the apparatus includes the measurement device 12 capable of measuring the fluid pressure in the closed circuit of the state of driving the dialysate infusing pump 13 in the test process, and the determination device 17 which determines whether or not the dialysate infusing line L8 is connected normally to the collection port 11 based on the measured value of the measurement device 12, it is possible to more accurately and automatically perform the connection confirmation of the dialysate infusing line L8 to the collection port 11.

Although the present invention has been described as above, the present invention is not limited thereto, but, for example, a negative pressure may be applied to the closed circuit by driving the dialysate infusing pump 13 for the forward rotation in the test process. Furthermore, the measurement device 12 for measuring the fluid pressure may be disposed in the closed circuit formed in the test process, and, for example, may be connected to the branch line L7 and the dialysate introduction line L1. Furthermore, in the present embodiment, although the other end of the dialysate infusing line L8 is connected to the arterial air trap chamber 5 of the arterial blood circuit 2 or the venous air trap chamber 6 of the venous blood circuit 3, the other end of the dialysate infusing line L8 may be connected to other parts of the arterial blood circuit 2 or the venous blood circuit 3.

### Industrial Applicability

If a blood purification apparatus is configured so that the closed circuit is formed in the flow route of the dialysate of the dialysate introduction line side including a predetermined part formed with the collection port, and the test process for the connection confirmation of the dialysate infusing line to the collection port is performed by measuring the fluid pressure in the closed circuit while driving the dialysate infusing pump, the apparatus can also be applied to blood purification apparatus with other added functions or the like.

### Reference Signs List

- 1: dialyzer (blood purifier)
- 2: arterial blood circuit
- 3: venous blood circuit
- 4: blood pump
- 5: arterial air trap chamber
- 6: venous air trap chamber
- 7: duplex pump
- 8, 9: filtration filter
- 10: ultrafiltration pump
- 11: collection port
- 12: measurement device
- 13: dialysate infusing pump
- 14: clamp device
- 15: control device
- 16: storage device
- 17: determination device
- 18: lid member
- 19: shaft
- 20: spring
- 21: magnet
- 22: lead switch
- 23: connector member
- L1: dialysate introduction line
- L2: dialysate discharge line
- L8: dialysate infusing line

## Claims

1. A blood purification apparatus comprising:
a blood purifier (1) which includes a blood purification membrane and performs the blood purification in the blood purification membrane;
an arterial blood circuit (2), a proximal end of which is connected to the blood purifier (1) and in which a blood pump (4) is disposed in the middle thereof;
a venous blood circuit (3), a proximal end of which is connected to the blood purifier (1);
a dialysate introduction line (L1) which introduces a dialysate into the blood purifier (1);
a dialysate discharge line (L2) which discharges the dialysate from the blood purifier (1);
a dialysate infusing line (L8), one end of which is connected to a collection port (11) formed in a predetermined part of the dialysate introduction line (L1), and the other end of which is connected to the arterial blood circuit (2) or the venous blood circuit (3); and
a dialysate infusing pump (13) which is disposed in the dialysate infusing line (L8) and is able to supply the dialysate of the dialysate introduction line (L1) to the arterial blood circuit (2) or the venous blood circuit (3),
wherein a closed circuit is formed in a flow route of the dialysate of the dialysate introduction line (L1) side including the predetermined part formed with the collection port (11), and a test process for confirming the connection of the dialysate infusing line (L8) to the collection port (11) by measuring a fluid pressure in the closed circuit while driving the dialysate infusing pump (13) is performed,
the blood purification apparatus further comprising:
a measurement device (12) capable of measuring a fluid pressure in the closed circuit of the state where the dialysate infusing pump (13) is driven in the test process;
a determination device (17) for determining whether or not the dialysate infusing line (L8) is connected normally to the collection port (11) based on a measured value of the measurement device (12); and
a control device (15) formed with a storage device (16) and the determination device (17), **characterised in that** the control device (15) is configured to perform the test process.

2. The blood purification apparatus according to claim 1,
wherein clamp device (14) capable of opening and closing the flow route thereof is disposed in the dialysate infusing line (L8), and the connection confirmation of the dialysate infusing line (L8) and the opening and closing confirmation of the clamp device (14) can be performed in the test process.

3. The blood purification apparatus according to claim 1 or 2,
wherein a positive pressure is applied to the closed circuit by driving the dialysate infusing pump (13) in reverse rotation in the test process.

## Patentansprüche

1. Blutreinigungsvorrichtung, aufweisend:
einen Blutreiniger (1), der eine Blutreinigungsmembran beinhaltet und die Blutreinigung in der Blutreinigungsmembran durchführt;
einen arteriellen Blutkreislauf (2), dessen proximales Ende mit dem Blutreiniger (1) verbunden ist und in dessen Mitte eine Blutpumpe (4) angeordnet ist;
einen venösen Blutkreislauf (3), dessen proximales Ende mit dem Blutreiniger (1) verbunden ist;
eine Dialysat-Eintragsleitung (L1), die ein Dialysat in den Blutreiniger (1) einbringt;
eine Dialysat-Austragsleitung (L2), die das Dialysat aus dem Blutreiniger (1) abführt;
eine Dialysat-Infusionsleitung (L8), deren eines Ende mit einer Sammelöffnung (11) verbunden ist, die in einem vorbestimmten Teil der Dialysat-Eintragsleitung (L1) ausgebildet ist, und deren anderes Ende mit dem arteriellen Blutkreislauf (2) oder dem venösen Blutkreislauf (3) verbunden ist; und
eine Dialysat-Infusionspumpe (13), die in der Dialysat-Infusionsleitung (8) angeordnet ist und fähig ist, das Dialysat von der Dialysat-Eintragsleitung (L1) dem arteriellen Blutkreislauf (2) oder dem venösen Blutkreislauf (3) zuzuführen,
wobei ein geschlossener Kreislauf in einem Strömungsweg des Dialysats aufseiten der Dialysat-Eintragsleitung (L1) ausgebildet ist, die den vorbestimmten Teil beinhaltet, welcher mit der Sammelöffnung (11) ausgebildet ist und ein Prüfprozess zur Bestätigung des Anschlusses der Dialysat-Infusionsleitung (L8) an die Sammelöffnung (11) durchgeführt wird, und zwar durch Messen eines Fluiddruckes in dem geschlossenen Kreislauf, während die Dialysat-Infusionspumpe (13) angetrieben wird,
wobei die Blutreinigungsvorrichtung weiter aufweist:
eine Messvorrichtung (12), die fähig ist, einen Fluiddruck in dem geschlossenen Kreislauf des Zustands zu messen, bei dem die Dialysat-Infusionspumpe bei dem Prüfprozess angetrieben wird;
eine Bestimmungsvorrichtung (17) zum Bestimmen, ob die Dialysat-Infusionsleitung (L8) in normaler Weise an die Sammelöffnung (11) angeschlossen ist oder nicht, und zwar basierend auf einem Messwert der Messvorrichtung (12); und
eine Steuervorrichtung (15), die mit einer Speichervorrichtung (16) und der Bestimmungsvorrichtung (17) ausgebildet ist, **dadurch gekennzeichnet, dass** die Steuervorrichtung (15) konfiguriert ist, den Prüfprozess durchzuführen.

2. Blutreinigungsvorrichtung nach Anspruch 1,
wobei eine Klemmvorrichtung (14), die fähig ist, den Strömungsweg zu öffnen und abzusperren, in der Dialysat-Infusionsleitung (L8) angeordnet ist, und die Bestätigung des Anschlusses der Dialysat-Infusionsleitung (L8) und die Bestätigung des Öffnens und Absperrens der Klemmvorrichtung (14) bei dem Prüfprozess durchgeführt werden kann.

3. Blutreinigungsvorrichtung nach Anspruch 1 oder 2,
wobei ein positiver Druck auf den geschlossenen Kreislauf aufgebracht werden kann, und zwar durch Antreiben der Dialysat-Infusionspumpe (13) in umgekehrter Drehrichtung beim Prüfprozess.

## Revendications

1. Appareil de purification de sang comprenant :
un purificateur de sang (1) qui comprend une membrane de purification de sang et réalise la purification de sang dans la membrane de purification de sang ;
un circuit de sang artériel (2), dont une extrémité proximale est raccordée au purificateur de sang. (1) et dans lequel une pompe de sang (4) est disposée dans son centre ;
un circuit de sang veineux (3), dont une extrémité proximale est raccordée au purificateur de sang (1) ;
une ligne d'introduction de dialysat (L1) qui introduit un dialysat dans le purificateur de sang (1) ;
une ligne de décharge de dialysat (L2) qui décharge le dialysat du purificateur de sang (1) ;
une ligne de perfusion de dialysat (L8), dont une extrémité est raccordée à un orifice de collecte (11) formé dans une partie prédéterminée de la ligne d'introduction de dialysat (L1), et dont l'autre extrémité est raccordée au circuit de sang artériel (2) ou au circuit de sang veineux (3) ; et
une pompe de perfusion de dialysat (13) qui est disposée dans la ligne de perfusion de dialysat (L8) et est apte à amener le dialysat de la ligne d'introduction de dialysat (L1) au circuit de sang artériel (2) ou au circuit de sang veineux (3),
dans lequel un circuit fermé est formé dans une voie d'écoulement du dialysat du côté de la ligne d'introduction de dialysat (L1) comprenant la partie prédéterminée formée avec l'orifice de collecte (11), et un processus de test pour confirmer le raccordement de la ligne de perfusion de dialysat (L8) à l'orifice de collecte (11) en mesurant une pression de fluide dans le circuit fermé tout en entraînant la pompe de perfusion de dialysat (13), est réalisé,
l'appareil de purification de sang comprenant en outre :
un dispositif de mesure (12) capable de mesurer une pression de fluide dans le circuit fermé de l'état dans lequel la pompe de perfusion de dialysat (13) est entraînée dans le processus de test ;
un dispositif de détermination (17) pour déterminer si la ligne de perfusion de dialysat (L8) est normalement raccordée à l'orifice de collecte (11) ou pas, en fonction d'une valeur mesurée du dispositif de mesure (12) ; et
un dispositif de commande (15) formé avec un dispositif de stockage (16) et le dispositif de détermination (17), **caractérisé en ce que** :
le dispositif de commande (15) est configuré pour réaliser le processus de test.

2. Appareil de purification de sang selon la revendication 1,
dans lequel un dispositif de pince (14) propre à ouvrir et fermer sa voie d'écoulement est disposé dans la ligne de perfusion de dialysat (L8) et la confirmation de raccordement de la ligne de perfusion de dialysat (L8) et la confirmation d'ouverture et de fermeture du dispositif de pince (14) peuvent être réalisées dans le processus de test.

3. Appareil de purification de sang selon la revendication 1 ou 2,
dans lequel une pression positive est appliquée sur le circuit fermé en entraînant la pompe de perfusion de dialysat (13) en rotation inverse lors du processus de test.
